# FASCICULE DE BREVET EUROPEEN

(11) **EP 1 232 283 B1**
(45) Date de publication et mention de la délivrance du brevet: **24.05.2006**
(21) Numéro de dépôt: 00974189.3
(22) Date de dépôt: 27.10.2000
(51) Int. Cl.: C12Q 1/68, G01N 33/50, G01N 33/543

(54) **Immuno PCR en temps réel utilisant un ADN-chimère comme marqueur d'amplification**
Immuno-Real-Time-PCR unter Verwendung eines DNA-chimers als Amplifikationsmarker
Immuno-real-time PCR using a chimeric DNA marker for amplification

(30) Priorité: 27.10.1999 BE 9900707; 14.03.2000 BE 200000196
(43) Date de publication de la demande: 21.08.2002
(73) Titulaire: Université de Liège, 4020 Liège (BE)
(72) Inventeur: ZORZI, Willy, B-5100 Seraing (BE); MELEN, Laurence, B-4920 Harzé (BE); ZORZI, Danièle, B-4680 Oupeye (BE); EL MOUALIJ, Benaissa, B-4020 Liège (BE); HEINEN, Ernst, B-4453 Juprelle (BE)
(74) Mandataire: Colens, Alain M.G.M.
(86) Numéro de dépôt international: PCT/BE2000/000131
(87) Numéro de publication internationale: WO 2001/031056

(56) Documents cités:
- WO-A-97/32044
- WO-A-98/20148
- WO-A-99/63109
- GB-A- 2 319 607
- CARSON RICHARD T ET AL: "Simultaneous quantitation of 15 cytokines using a multiplexed flow cytometric assay." JOURNAL OF IMMUNOLOGICAL METHODS, vol. 227, no. 1-2, 30 juillet 1999 (1999-07-30), pages 41-52, XP004178041 ISSN: 0022-1759
- SANO T ET AL: "Deoxyribonucleic acids as unique markers in molecular detection" GENETIC ANALYSIS: BIOMOLECULAR ENGINEERING,US,ELSEVIER SCIENCE PUBLISHING, vol. 14, no. 2, 1 juillet 1997 (1997-07-01), pages 37-40, XP004126263 ISSN: 1050-3862
- HENDRICKSON EDWIN R ET AL: "High sensitivity multianalyte immunoassay using covalent DNA-labeled antibodies and polymerase chain reaction." NUCLEIC ACIDS RESEARCH, vol. 23, no. 3, 1995, pages 522-529, XP002151883 ISSN: 0305-1048
- DATABASE BIOSIS [en ligne] BIOSCIENCES INFORMATION SERVICE, PHILADELPHIA, PA, US; 1996 SCHOEN C D ET AL: "Detection of potato leafroll virus in dormant potato tubers by immunocapture and a fluorogenic 5' nuclease RT-PCR assay." Database accession no. PREV199699190638 XP002172198 & PHYTOPATHOLOGY, vol. 86, no. 9, 1996, pages 993-999, ISSN: 0031-949X
- SIMS P.W. ET AL: 'Immunopolymerase chain reaction using real-time polymerase schain reaction for detection' ANALYTICAL BIOCHEMISTRY vol. 281, no. 2, 01 Juin 2000, ACADEMIC PRESS, SAN DIEGO, CA, US, pages 230 - 232, XP001147707

## Description

La présente invention se rapporte à une méthode de détection par PCR, notamment par immuno-PCR, d'entités microbiologiques et en particulier d'éléments ou de molécules cibles.

L'immuno-PCR est une technique connue qui est décrite, pour certaines applications, par exemple dans SANO et al Science (1992) 258:120-2, Case et al. Journal of Immunological Methods (1999) 223:93-106, KAKIZAKI et al. Letters in Applied Microbiology (1996); 23(2); 101-103, Taylor et al Veterinary Microbiology (1997) 56:135-145, DOCHERTY et al. Letters in Applied Microbiology (1996) 22:288-292.

Par ailleurs, on connaît la méthode ELISA qui est une technique aussi spécifique mais moins sensible d'un facteur 10² à 10⁵ par rapport à l'immuno-PCR.

Plus récemment, des techniques et des appareils de PCR dite quantitative sont apparus. Ces appareils détectent et mesurent un signal, par exemple de fluorescence, représentatif d'une quantité d'amplicons spécifiques du fragment d'ADN, marqué au cours de la PCR par un agent intercalant de l'ADN excitable par un rayonnement ultra-violet. La caractéristique de cette technique est que la mesure est effectuée en temps réel c'est à dire durant et non après la phase exponentielle, de préférence mais pas nécessairement par comparaison avec un témoin. Cette technique s'avère supérieure par maints aspects aux techniques de PCR compétitive par co-amplification.

A la connaissance du demandeur, quatre appareils de PCR quantitative sont actuellement distribués en Europe: le LightCycler de la firme Roche, le GeneAmp 5700 et le GeneAmp 7700 Sequence Détection System de la firme PE Applied Biosystems et le icycler iQ de la firme Bio-Rad.

Selon l'invention, on a constaté que, de manière surprenante, la technique d'immuno-PCR peut être avantageusement appliquée avec un appareil de PCR quantitative.

Selon l'invention, on a en effet constaté qu'il est possible de fixer des anticorps sur les parois de microtubes PCR destinés ou adaptés à ces appareils, en particulier si ces parois sont en plastique résistant aux cycles de chauffage de la PCR.

On a constaté de plus que la fixation des anticorps de capture sur les parois des microtubes PCR et donc leur présence dans le milieu durant l'analyse sous forme de protéines dénaturées ou non n'interfère pas de manière substantielle avec la lecture du signal d'amplification par le détecteur de fluorescence d'un appareil d'immuno-PCR quantitative.

Il en est de même pour l'ajout de l'antigène ainsi que des autres constituants de l'immuno-PCR qui n'interfèrent pas de façon substantielle au niveau de l'efficacité du processus de la PCR.

De bons résultats ont été obtenus en utilisant des tubes en polypropylène tels qu'utilisables avec un appareil 5700 ou 7700 Sequence Detection System de PE Applied Biosystems.

Ces constatations ont mené, selon un premier aspect de l'invention, à la mise au point d'une nouvelle méthode de détection et de dosage d'éléments antigéniques, par immuno-PCR quantitative(iPCRq).

Selon un autre aspect de l'invention, on propose l'application de la PCR quantitative à la détection et la quantification d'éléments ou molécules cibles, éventuellement non antigéniques, par association de ceux-ci avec des "ligands". Les ligands (par ex un peptide ou une hormone) sont, d'une part, couplés directement ou indirectement à l'acide nucléique "reporter" (ou « marqueur ») et, d'autre part, les molécules ou éléments cibles sont fixables sur les parois des tubes PCR ou de billes, p.e. des billes magnétiques ou de latex, ou encore sur des membranes, la fixation étant éventuellement effectuée indirectement, par exemple par l'intermédiaire d'un anticorps de capture. Ainsi, selon cette variante, on a remplacé l'anticorps de capture par un ligand ou un récepteur. Les couples ligands-molécules cibles fonctionnent comme les couples anticorps-antigènes.

L'invention sera avantageusement appliquée pour la détection et/ou le dosage de différentes entités biologiques principalement en analyse clinique. Elle apporte effectivement une simplification du procédé de dosage par immuno-PCR tout en présentant une possibilité de quantification rapide et un gain économique appréciable. L'immuno-PCR selon l'invention se prête bien à une automatisation de sa procédure.

De manière générale, l'invention concerne un procédé de détection d'un élément A tel que définie dans les revendications
a. comprenant les étapes suivantes:
   - une étape de fixation dudit élément A, directement ou par l'intermédiaire d'un agent de capture, à une ou des surfaces à l'intérieur du ou des récipients d'un appareil de mesure PCR,
   - une étape d'association audit élément A d'un système de couplage à un acide nucléique marqueur
   - une étape d'amplification de l'acide nucléique marqueur
b. caractérisé en ce que les mesures sont effectuées durant la phase d'amplification de l'acide nucléique marqueur.

La détection quantitative selon la présente invention peut être avantageusement réalisée avec un appareil GeneAmp 5700 ou 7700 Sequence Détection System de PE Applied Biosystems(voir Everett K.D.E.) J. Clin. Microbiol. (1999), 37: 575-580. La fluorescence des échantillons est comparée à celle de témoins durant la phase exponentielle de la PCR, et non au niveau des plateaux comme c'est le cas pour les protocoles de détection classiques en immuno-PCR.

L'invention tel que définie dans les revendications se rapporte donc, notamment, à un procédé de détection par immuno-PCR d'un élément antigénique, le procédé comprenant les étapes suivantes :
- on couvre une surface d'un récipient ou d'une bille avec une solution d'un anticorps monoclonal ou polyclonal
- on ajoute une solution de l'élément antigénique comportant au moins deux sites antigéniques dont l'un au moins complémentaire audit anticorps
- on associe un fragment d'ADN à au moins un second anticorps, de préférence monoclonal, pour obtenir un conjugué
- on ajoute le conjugué ADN/anticorps de préférence monoclonal à la solution
- on réalise une PCR sur les fragments d'ADN ainsi immobilisés
- on détecte et mesure l'amplification, durant la phase exponentielle, par exemple par fluorescence.

En effet, le fait d'avoir obtenu après amplification un signal fluorescent proportionnel à la quantité d'antigène introduite dans le système (par exemple coefficient de corrélation des différents points de la courbe standard = 98.66%) démontre que cette nouvelle technique est capable de détecter et de quantifier la présence de faibles quantités d'antigènes dans un milieu.

Les éléments antigéniques détectables et quantifiables par la méthode selon l'invention sont variés. A priori, tout antigène pouvant être détecté et dosé par l'immuno-PCR classique ou par la méthode ELISA peut l'être par le procédé d'immuno-PCR quantitative de la présente invention.

De manière non exhaustive, selon l'invention,
on peut citer la détection de :
- protéines (ex: sérumalbumine bovine, voir p.e. Sano et al, Science, 1992, 258, 120-122);
- antigènes de surface (ex: virus de l'Herpes Simplex, voir Mweene et al, J. Clin. Microbiol., 1996, 34, 748-750; virus de l'Hépatite B, Niemeyer et al, Analytical Biochem., 1997, 246, 140-145);
- carbohydrates (ex: 06-méthylguanosine, Case et al, J. Immunol. Methods, 1999, 223, 93-106; ganglioside membranaire GD2, Tsai et al, JNCI, 1984, 73, 627-633; ganglioside GM3, Zhang et al, American J. of Pathology, 1998, 152, 1427-1432),
- hormones (ex: hTSH et hCG, Joerger et al, Clin. Chem., 1995, 41, 1371-1377),
- enzymes (ex: Beta-galactosidase, Hendrickson et al, Nucl. Acids Research, 1995, 23, 522-529; β-glucuronidase, Chang et al, J. Immunol.Methods, 1997, 208, 35-42),
- bactéries (ex: Pasteurella piscicida, Kakizaki et al, Letters in Applied Microbiol, 1996, 23, 101-103),
- ligands ou récepteurs (ex: IAM-1 (molécule d'adhésion intercellulaire aux récepteurs leucocytaires LFA-1 et Mac-1), Itoh et al, Artificial Organs, 1996, 20, 898-901; récepteurs de lymphocytes T interagissant avec les complexes antigéniques MHC, Sperl et al, J. Immunol. Methods, 1995, 186, 181-194),
- composés organiques (ex: benzopyrène diol-époxyde guanine, Denissenko et al, Biotechniques, 1996, 21, 187-188),
- antigènes tumoraux (ex: proto-oncogène humain ETS-1, Zhou et al, Nucl. Acids Res., 1993, 21, 6038-6039; ganglioside GM3, Zhang et al, American J. of Pathology, 1998, 152, 1427-1432),
- peptides (ex: alpha-human atrial natriuretic peptide, Numata et al, Clinica Chimica Acta, 1997, 259, 169-176 ; human interleukin-18, Furuya et al., J Immunol Methods. 2000 21;238(1-2):173-80; angiotensinogen, Sugawara et al., Clin Chim Acta. 2000;299(1-2):45-54).
- molécules de prolifération cellulaire (ex: thiorédoxine, Das et al, J. Immunol. Methods, 1998, 211, 9-20).
- cytokines (ex: TNF-alpha, Sanna et al, Proc. Nat. Acad. Sci. USA, 1995, 92, 272-275).

Toutes ces références indiquent la possibilité d'adapter un système de détection ELISA à une détection par immuno-PCR. Tout système de détection d'une molécule déjà réalisée par ELISA peut donc être transposé à l'immuno-PCR , ceci étant valable notamment aussi pour les systèmes suivants:
- Détection d'agents infectieux (ex: protéine prp, Matsushita et al, J. Vet. Med. Sci., 1998, 6, 777-779).
- Détection de composés aromatiques (ex: dioxines/furanes, Immunoassay Kit, CAPE Technologies, LLC, South Portland, USA) .

Selon un autre aspect de l'invention, l'immuno-PCR quantitative a été rendue applicable à la détection de molécules antigéniques extraites d'organes ou de matrices complexes.

L'invention a ainsi été également appliquée à la détection de peptides et de protéines prions. Il s'agit d'une adaptation immuno-PCR selon l'invention d'un système ELISA qui a été au préalable développé par nos soins, appliquée à la détection de séquences polypeptidiques de protéines prions murines et bovines normales et infectieuses.

L'application de l'iPCRq à la détection de prions infectieux améliore le dépistage des animaux contaminés et ce à des stades précoces de l'infection par les maladies à prion. Ceci permet d'appliquer des traitements adaptés ou de sacrifier les bêtes.

On suspecte également depuis peu une transmission au bétail via l'environnement et via le fourrage (transmission possible via la bouse des bovins malades qui pourrait contaminer les pâturages à long terme). La technique proposée par l'invention permet donc de détecter les prions infectieux présents à l'état de trace dans les fourrages et dans les matrices environnementales telles que les champs de pâturage où ont stationné des animaux infectés.

Il existe actuellement trois tests de détection des protéines prions qui sont reconnus par l'Union Européenne à savoir ceux de Prionics, Enfer Scientific et Biorad. Toutefois ces tests manquent de sensibilité.

Le schéma expérimental de détection peut faire appel aux mêmes étapes que celles décrites ci-après dans le cas de l'iPCRq appliqué à la détection de l'hIL-4.

Selon un aspect de l'invention, l'élément à doser est plus particulièrement un ou (des) polypeptide(s) prion(s) infectieux, qui peut être présent dans un extrait de matrice complexe organique ou environnementale.

Plus généralement l'élément à doser est un ou (des) polypeptide(s) prion(s) :
- de synthèse, de forme recombinante produit-(s)artificiellement par génie génétique et/ou
- natif(s), issu(s) d'extraits de matrices complexes organiques et/ou environnementales et/ou
- non purifié(s) ou purifié(s) partiellement ou totalement et/ou
- sain(s) ou infectieux et/ou
- de séquence partielle ou totale par rapport à la séquence de la protéine et/ou
- résistant(s) ou non résistant(s) à la protéinase K et/ou
- d'origine cellulaire ou exogène et/ou
- en solution pure ou en solution complexe ou sur support.

L'invention est basée sur l'utilisation d'un fragment d'ADN chimérique (ADN Marqueur) et des amorces particulièrement adaptées pour le procédé d'immuno-PCR susmentionné.

L'invention est également applicable à la détection d'éléments antigéniques, différents ou non, ou autres en multiplex de façon concomitante.

Afin d'élargir le spectre d'application de la technique d'immuno-PCR quantitative, on propose en effet une technique de détection concomitante en multiplex d'antigènes différents ou non utilisant des anticorps ou d'autres molécules couplés à des sondes ADN marqueurs. Lors de PCR en multiplex, les sondes marqueurs doivent avoir des sites d'hybridation aux amorces nucléotidiques différents à au moins une base près.

Ce couplage peut être direct , chimique ou enzymatique ou indirect c'est-à-dire effectué par l'intermédiaire d'une molécule « spacer » pouvant être une protéine, un polypeptide, un groupement aromatique, un composé organique ou un composé inorganique. L'ADN ainsi fixé servira de matrice pour le processus d'amplification par PCR.

La séquence nucléotidique de l'ADN "marqueur" utilisée sera modifiée de telle sorte que les sondes oligonucléotidiques marquées telles les sondes Taqman™, scorpio™ , "molecular beacons" (Eurogentec) permettent sur base du composé fluororescent utilisé et émettant à des longueurs d'ondes différentes une détection simultanée de plusieurs antigènes. Ces sondes peuvent
- être couplées à des fluorophores tels que FAM, HEX, TET, CY3, CY5, C5.5, JOE, 6-ROX, Cascade Blue, Texas Red, Rhodamine, Rhodamine green, Rhodamine red, Rhodamine 6G, 6-TAMRA, 5-TMRIA, Alexa 430, Alexa 488, Alexa 594, Bodipy R6G (Eurogentec) ou
- dans le cas des sondes Taqman™, être utilisées avec le quencher TAMRA et les fluorophores FAM, HEX ou TET.

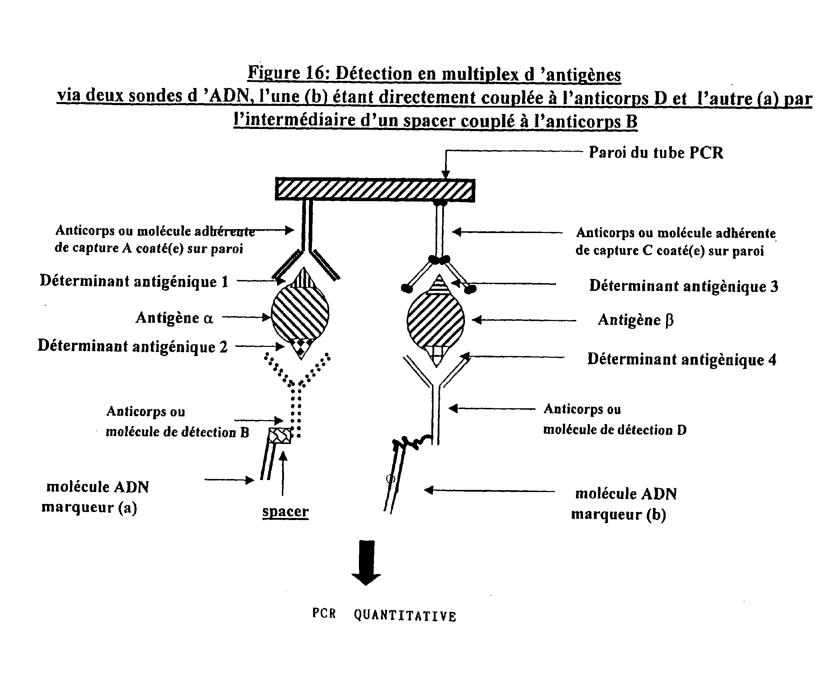

Les exemples suivants illustrent de manière non limitative l'invention et se réfèrent aux figures 1 à 15 , dans lesquelles
- la fig. 1 est un graphique illustrant l'intensité de la fluorescence en fonction du nombre de cycle (courbes d'amplification), pour différents puits pour une expérience de détection de l'interleukine humaine Il-4
- la fig. 2 est le graphique correspondant reprenant les valeurs de Ct en fonction de la position des puits
- la fig. 3 est le graphique montrant la courbe standard résultante de la fig 1
- la fig. 4 illustre l'effet des agents de saturation sur l'immuno-PCR appliqué à des systèmes identiques à celui illustré dans les figs 1 à 3
- la fig. 5 illustre l'effet des supports (microtubes) sur l'immuno-PCR appliqué à des systèmes identiques à celui illustré dans les figs. 1 à 3
- la fig. 6 représente la courbe standard pour la détection par immuno-PCR de la digoxigénine
- les figs. 7 , 8 et 9 illustrent la détection quantitative de la biotine dans le système ligand-PCR
- la fig. 10 illustre à titre comparatif un test ELISA sur un fragment de peptide prion
- les figs. 11 et 12 illustrent les courbes d'intensité de fluorescence et de Ct obtenues en immuno-PCR selon l'invention pour le même fragment de peptide prion
- la fig. 13 illustre à titre comparatif un test ELISA sur protéines prions extraits à partir cerveau de boeuf
- la fig. 14 illustre les Cₜ obtenus lors de la détection de prions dans une matrice complexe à savoir un extrait de cerveau de boeuf
- la fig.15 illustre un ELISA sur protéines prions extraits à partir de cerveau de boeuf avec et sans protéinase K.

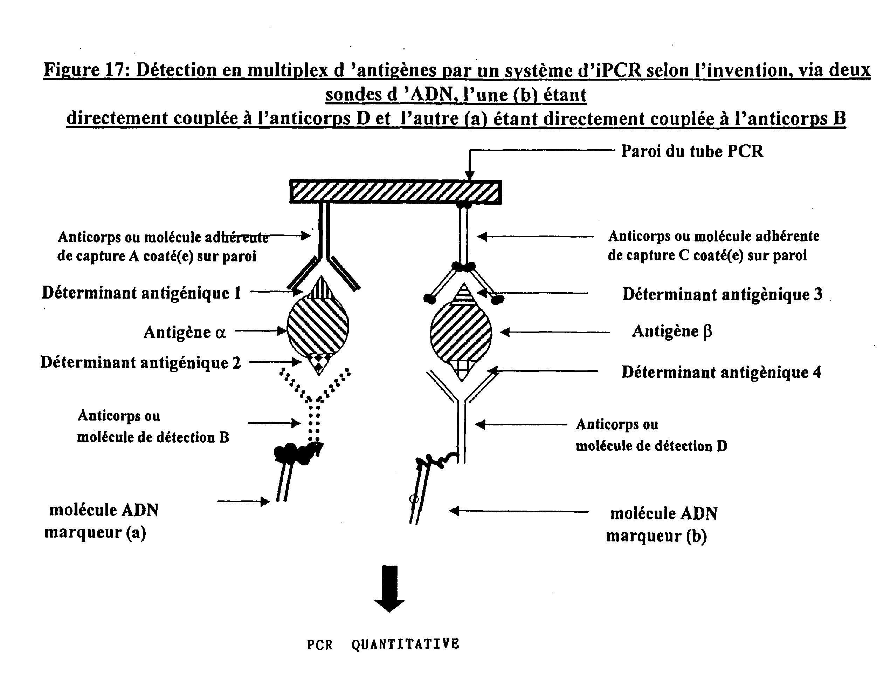

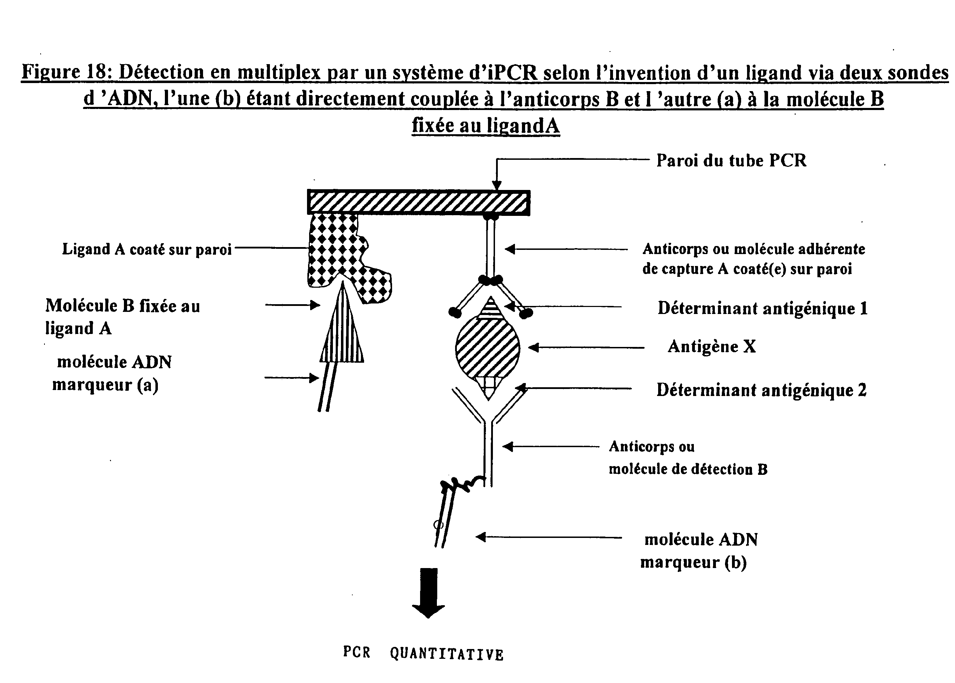

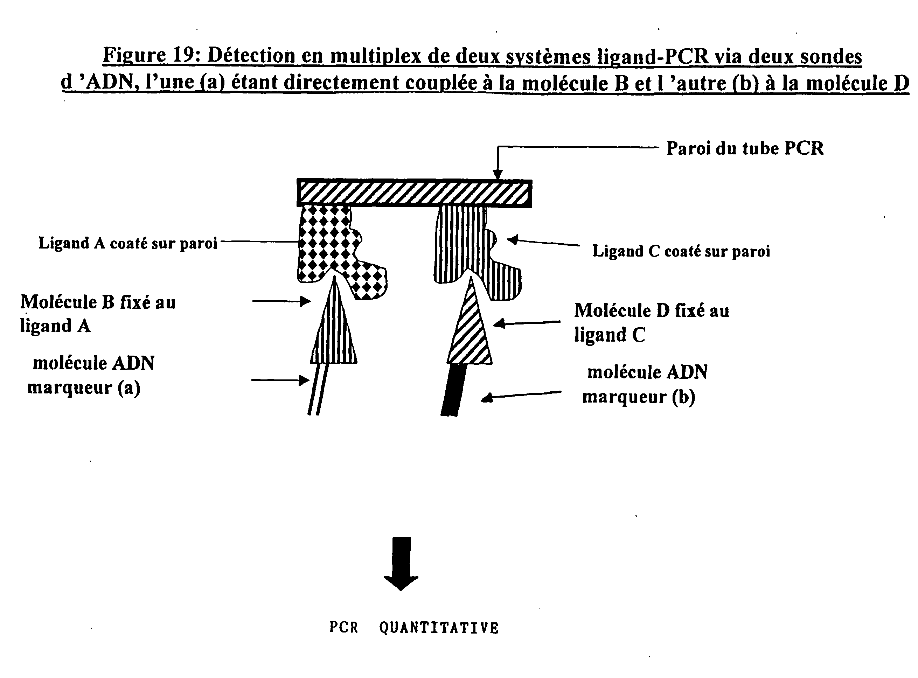

- les figs. 16 à 19 sont des schémas illustrant la détection en multiplex par PCR quantitative. Les figs. 16 et 17 représentent deux schémas illustrant le principe de la détection en multiplex par PCR quantitative en utilisant un anticorps couplé directement à l'ADN marqueur , ou couplé via un « spacer ». Les figs. 18 et 19 représentent deux schémas illustrant la détection en multiplex d'un antigène et d'un ligand via deux sondes d'ADN.

### EXEMPLE 1 - INTERLEUKINE HUMAINE Il-4

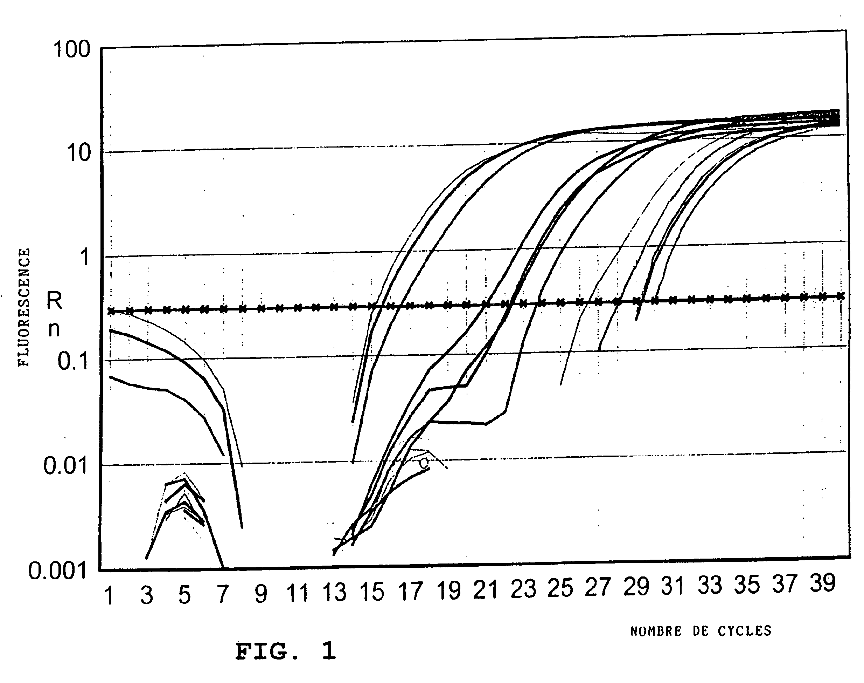

Une mise au point de la technique d'immuno-PCR quantitative a été réalisée à titre d'exemple non limitatif et en se référant aux figures 1 à 5, sur base d'une méthode de détection immunologique de l'interleukine humaine Il-4 (hIL-4).

Le procédé décrit à titre illustratif comprend les étapes suivantes:
1- obtention d'un anticorps monoclonal de capture dirigé contre hIL-4 obtenu dans le commerce (Biosource) (référence :AHC0642)
2- fixation de cet anticorps dans une plaque optique à 96 puits (PE Applied Biosystems), en tampon de coatage (NaHCO₃ 0.05M,
   Na2CO3 0.05M, pH 9.4) à 4°C pendant 16 heures, à une concentration de 1 µg/ml (50 µl/puits)
3- 4 lavages en solution de lavage (NaCl 0.15M, Tween 20 0.1%, pH 7.4) (300 µl/puits)
4- Saturation des sites non saturés par l'anticorps de capture en tampon de saturation (NaCl 0.14M, Na2HPO4.2H2O 8 mM, KH2PO4 1.5 mM, KCl 2.7 mM, BSA 3%, pH 7.4) pendant 2 heures à température ambiante (200 µl/puits)
5- 5 lavages en solution de lavage
6- ajout de l'antigène à capturer (50 µl/puits) en tampon de dilution (NaCl 0.14M, Na2HPO4.2H2O 8 mM, KH2PO4 1.5 mM, KCl 2.7 mM, BSA 0.5%, Tween 20 0.1%, pH 7.4), aux concentrations suivantes : 5 pg/ml, 50 pg/ml, 500 pg/ml, 2.5 ng/ml, 5 ng/ml, 50 ng/ml, 500 ng/ml, 2.5 µg/ml, puis ajout d'un anticorps monoclonal biotinylé de détection dirigé contre hIL-4 (25 µl/puits), obtenu par le commerce (Biosource) (référence : AHC0749), à une concentration de 0.4 µg/ml en tampon de dilution, pendant 2 heures à température ambiante, sous une agitation constante de 280 rpm
7- 10 lavages en solution de lavage
8- obtention en parallèle d'une molécule d'ADN chimère biotylinée (DNA Reporter ou marqueur)
9- préparation en parallèle de streptavidine conjuguée dans le rapport molaire Streptavidine/DNA Reporter Biotinylé 0,5/1 (17 amoles/34 amoles), laissant ainsi deux sites libres pour la fixation de l'anticorps biotinylé
10- ajout de la streptavidine conjuguée dans ladite plaque, le rapport Strept/Ac B choisi est de 0,5/1, soit deux molécules d'Ac fixées par molécule de Streptavidine, pendant 30 minutes à température ambiante (50 µl/puits)
11- dix lavages en tampon de lavage suivis de 5 lavages en H20
12- PCR avec amorces spécifiques (SEQ ID NO :4 et 5) à l'ADN chimère dans un volume de 50µl composé de 25 µl d'une solution comprenant l'antigène à détecter et les amorces oligonucléotidiques et de 25 µl d'une solution « Mix-PCR » (PE Applied Biosystems, ref: 4309155) . La mesure quantitative est réalisée avec un appareil GeneAmp 5700 de PE Applied Biosystems en utilisant ladite plaque de 96 puits.

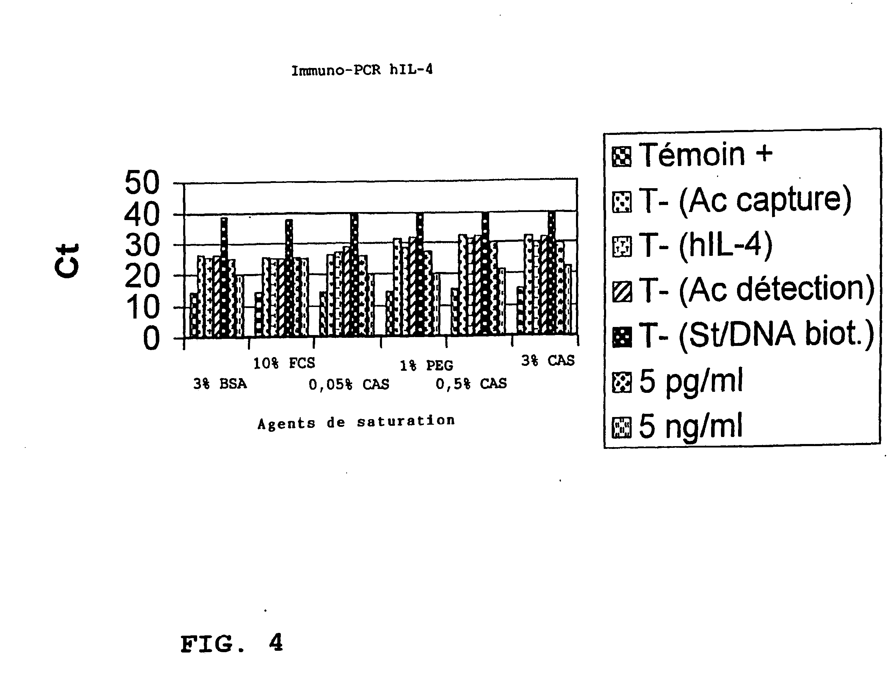

Le seuil de sensibilité de la méthode utilisée est comparable à celui d'un test ELISA réalisé sur l'hIL-4 en présence des anticorps monoclonaux de capture et de détection décrits dans le protocole ci-dessus (la détection est dans ce cas réalisée par ajout de streptavidine marquée à la peroxydase suivi d'une révélation colorimétrique). Par contre, cette méthode permet de quantifier des concentrations d'antigène comprises entre 5 pg/ml et 2.5 µg/ml, alors que les concentrations détectées par l'ELISA varient entre 5 pg/ml et 1 ng/ml. Dans une expérience similaire, (fig.4), on arrive à détecter, par ce système d'immuno-PCR selon l'invention, jusqu'à 5 femtog/ml d'hIL-4, indiquant une sensibilité 1000 fois supérieure à l'ELISA.

### Obtention de la molécule d'ADN marqueur biotinylée :

De préférence l'ADN chimère marqueur utilisé dans l'invention ne doit présenter, d'une part, aucune identité de séquence avec le matériel génétique de l'échantillon ou du milieu contenant l'antigène à détecter et d'autre part, être la plus éloignée possible de séquences existantes (procaryotes ou eucaryotes) susceptible de se trouver comme contaminant. Une PCR a été réalisée à partir du plasmide pHHL23 , plasmide recombinant constitué d'une séquence ARS (séquence eucaryote codant pour un anticorps de souris) clonée dans le vecteur pCRII-Topo (InVitroGen). Les amorces utilisées pour réaliser la PCR s'hybrident d'une part au niveau du Multi Cloning Site du vecteur pCRII-Topo (séquence de sites de restriction enzymatique successifs) et d'autre part dans la séquence ARS. Après 35 cycles de PCR (3 min à 96°C, 1 min à 94°C, 1 min à 66°C, 30 sec à 72°C, suivis d'une polymérisation de fin de cycles de 5 min à 72°C) en présence de l'amorce 1 Forward 5' biotinylée de séquence 5'Biotine-TATCCAATCCATTCCAGGCCC 3' (désignée dans l'invention SEQ ID NO :2) et de l'amorce 2 Reverse non biotinylée 5' TAA CGC CAG GGT TTT CCC AGT 3'(désignée dans l'invention SEQ ID NO :3), on obtient un produit de 246 pb de séquence suivante :
5'TATCCAATCCATTCCAGGCCCTGTCCAGGCCTCTGTTTCACCCAGTTTATACCGTAGCTTG TGAATGTATATCCAGAAGCCTTGCAGGACATCTTCACTGAGGACAAGGGCGAATTCTGCAGAT ATCCATCACACTGGCGGCCGCTCGAGCATGCATCTAGAGGGCCCAATTCGCCCTATAGTGAGT CGTATTACAATTCACTGGCCGTCGTTTTACAACGTCGTGACTGGGAAAACCCTGGCGTTA
3'(désignée dans l'invention SEQ ID NO :1) purifié sur gel d'agarose 2% (kit d'élution de l'ADN à partir de gels d'agarose de la firme Qiagen).

Afin d'amplifier le DNA marqueur biotinylé décrit ci-dessus, on utilise deux amorces non biotinylées internes à ce DNA Reporter ; il s'agit de l'amorce 3 Forward 5' GAA GCC TTG CAG GAC ATC TTC A 3' (désignée dans l'invention SEQ ID NO :4) et de l'amorce 4 Reverse 5' GCC GCC AGT GTG ATG GAT AT 3'(désignée dans l'invention SEQ ID NO :5). Après 40 cycles d'amplification (10 min à 95°C ; 15 sec à 95°C, 1 min à 60°C), on obtient un fragment de 67 pb analysé par le programme GeneAmp SDS software de la firme PE Applied Biosystems.

La figure 1 illustre les courbes d'amplification qui ont été obtenues lors des expériences de détection d'hIL-4 par immuno-PCR quantitative à l'aide du GeneAmp 5700 Sequence Détection System de la firme PE Applied Biosystems.
Ces courbes représentent la fluorescence (Rn) détectée au sein de chaque puits en fonction du nombre de cycles d'amplification. La fluorescence renseigne sur l'efficacité de la PCR.

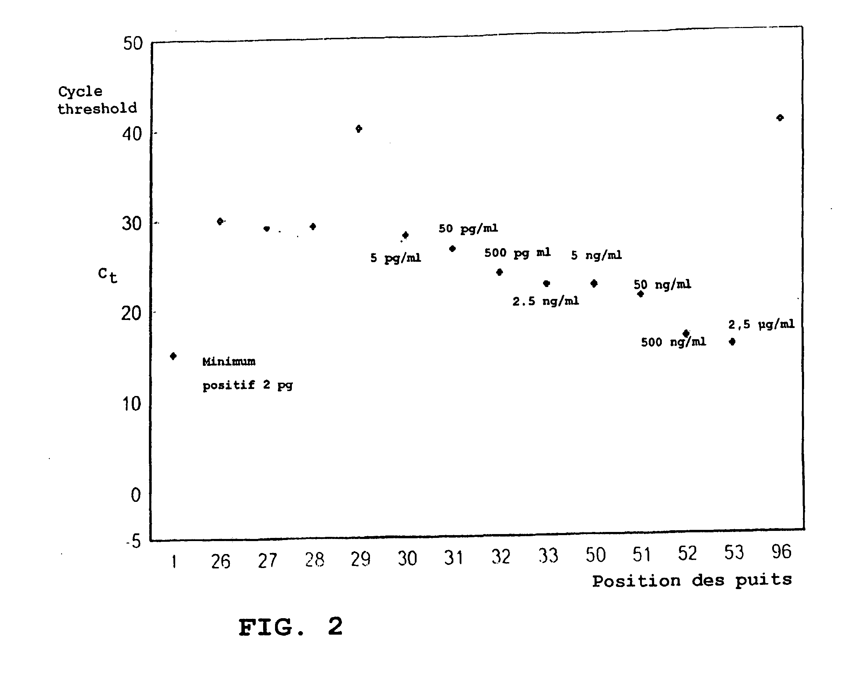

Après avoir établi une ligne de base pour les courbes d'amplification (en début de phase exponentielle), la figure 2 a été réalisée: elle représente le "Ct" (ou "Cycle Threshold", nombre de cycles pour lequel la courbe d'amplification de l'échantillon croise la ligne de base) en fonction de la position de l'échantillon dans la plaque (numéro du puits).

Les valeurs de Ct sont représentatives de la sensibilité de la PCR: un Ct faible signifie que le nombre de copies initial d'ADN marqueur était élevé et par conséquent que, la quantité d'antigènes immobilisés sur la plaque était élevée.

Les échantillons sont distribués comme suit:
- puits 1: témoin positif (ajout de 2 pg (12.5 attomoles) d'ADN marqueur biotinylé avant la PCR quantitative)
- puits 26: témoin sans ajout d'anticorps monoclonal de capture
- puits 27: témoin sans ajout d'hIL-4
- puits 28: témoin sans ajout d'anticorps monoclonal de détection
- puits 29: témoin sans ajout de complexe streptavidine/ADN Reporter biotinylé
- puits 30: échantillon testé en présence de 5 pg/ml d'hIL-4.
- puits 31: échantillon testé en présence de 50 pg/ml d'hIL-4
- puits 32: échantillon testé en présence de 500 pg/ml d'hIL-4
- puits 33: échantillon testé en présence de 2.5 ng/ml d'hIL-4
- puits 50: échantillon testé en présence de 5 ng/ml d'hIL-4
- puits 51: échantillon testé en présence de 50 ng/ml d'hIL-4
- puits 52: échantillon testé en présence de 500 ng/ml d'hIL-4
- puits 53: échantillon testé en présence de 2.5 µg/ml d'hIL-4
- puits 96: témoin négatif obtenu en l'absence de matrice d'ADN (mélange des réactifs de la PCR et des amorces)

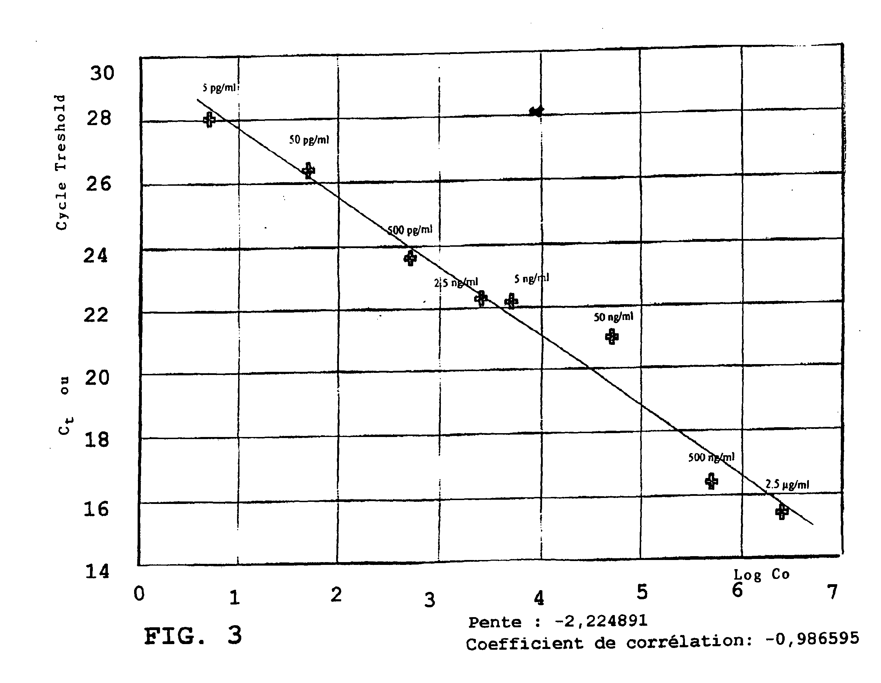

La figure 3 représente la courbe standard, obtenue en reportant les valeurs de Ct obtenues en fonction des concentrations d'hIL-4 testées.

Plusieurs autres expériences de détection d'hIL-4 par immuno-PCR quantitative ont été réalisées dans les plaques optiques PE Applied Biosystems à 96-puits (réf. N801-0560) en présence de divers agents saturants utilisés dans l'étape de bloquage des sites non saturés par l'anticorps monoclonal de capture et destinés à réduire le niveau de signal non spécifique obtenu pour les témoins négatifs suivants :-Anticorps monoclonal de capture, -hIL-4,-Anticorps monoclonal de détection (voir figure 2). Les résultats de ces expériences sont présentés à la figure 4. Lors de chaque expérience, deux concentrations d'antigène (5 pg/ml et 5 ng/ml) ont également été testées. Les résultats que nous avons obtenus en présence de PEG (polyéthylèneglycol) 1% se sont avérés identiques à ceux obtenus en présence de BSA 3% (agent bloquant utilisé dans le protocole de détection d'hIL-4 par immuno-PCR quantitative décrit précédemment); la présence de FCS (Fetal Calf Serum) 10% dans le tampon de saturation n'a par contre pas donné de bons résultats (valeurs de Ct identiques pour les deux concentrations d'antigène, et donc absence de quantification de cet antigène).

Le niveau du signal non spécifique s'est par contre révélé plus faible lors de l'utilisation de caséine 0,05, 0,5 et 3% dans le tampon de saturation (Ct des témoins négatifs -Ac capture, -hIL-4, -Ac détection nettement plus élevés que pour la condition BSA 3%) ; cependant, il n'a pas été observé de différences significatives entre les Ct de ces témoins et le Ct de la concentration d'antigène 5 pg/ml en présence de caséine, ce qui signifie que le seuil de sensibilité de l'immuno-PCR quantitative reste identique à celui observé en présence de BSA 3%.

Par ailleurs, des expériences de détection et de quantification d'hIL-4 par immuno-PCR quantitative ont également été réalisées dans des supports en polypropylène provenant de firmes autres que PE Applied Biosystems: plaques 96-puits de la firme Stratagene (réf. 410088), utilisées avec les "Optical Caps"(capuchons que l'on place à la surface des puits avant de réaliser la PCR et permettant la lecture optique) de la firme PE Applied Biosystems(réf. N801-0935); plaques 96-puits de la firme ABgene (Westburg, réf. AB-0900), utilisées avec des "Microcaps" de réf. AB-0602; strips de 8 tubes (assemblages de 8 tubes) de la firme Biozym (réf. 179302), utilisés avec des "caps" de réf. 179701.

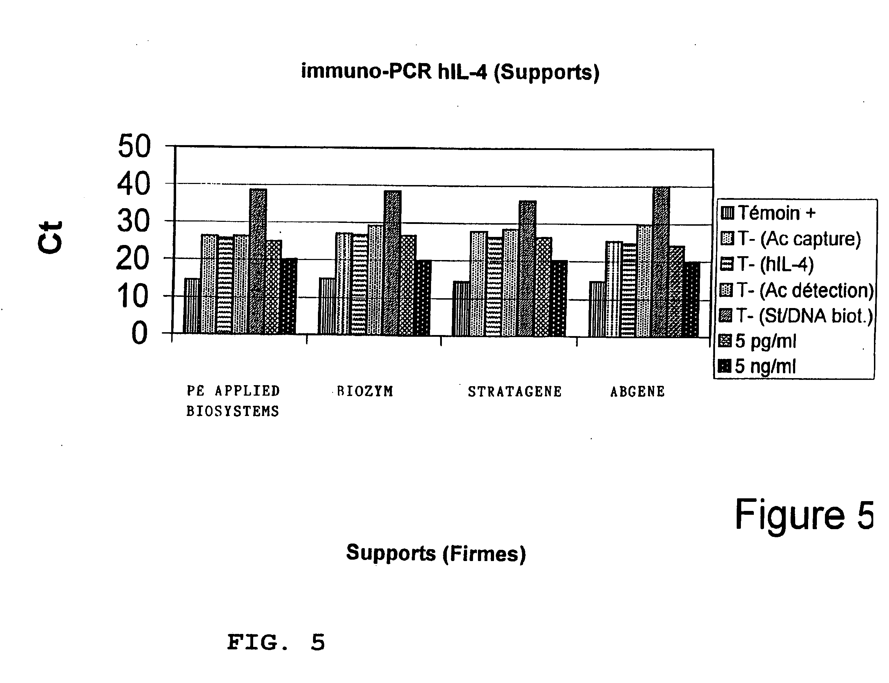

De même que pour les expériences concernant l'effet de divers agents bloquants sur le signal non spécifique, deux concentrations d'antigène (5 pg/ml et 5 ng/ml) on été testées pour chaque type de support. Les résultats obtenus sont présentés à la figure 5. Ils montrent que la détection et la quantification d'hIL-4 sont réalisables également à l'aide d'autres supports que ceux provenant de la firme PE Applied Biosystems.

### EXEMPLE 2 - Digoxigénine

Le procédé selon l'invention a également été testé en utilisant comme élément antigénique une molécule d'ADN marquée à la digoxigénine (DIG), et en adoptant la procédure suivante :
1- obtention d'un anticorps polyclonal contre la DIG disponible dans le commerce (Boehringer Mannheim, réf. 1 333 089)
2- "coatage" de cet anticorps dans une plaque optique à 96 puits (PE Applied Biosystems), en tampon hydrogénocarbonate (NaHC03 0.1M, pH 8.6) à 4°C pendant 16 heures, à raison de 2 µg/ml (50 µl/puits)
3- Saturation des sites non saturés par l'anticorps polyclonal en tampon PBS/5% BSA pendant 2 heures à 37°C (200 µl/puits)
4- 10 lavages en tampon PBST (Phosphate Buffered Saline + 0.05% Tween 20) (300 µl/puits)
5- capture de l'antigène pendant 1 heure à température ambiante, celui-ci étant obtenu en réalisant une PCR sur de l'ADN du phage lambda en présence de nucléotides DIG-11-dUTP produisant un fragment de 1400 pb marqué à la DIG (3 min à 96°C; 30 cycles de 30 sec à 94°C, 30 sec à 60°C, 1 min 40 sec à 72°C; élongation finale de 5 min à 72°C), l'antigène étant ajouté dans une solution PBS/1% BSA, à raison de 2.5 -25-125-1250-2500 et 4000 ng/ml (50 µl/puits)
6- 10 lavages en tampon PBST
7- ajout dans ladite plaque d'un anticorps secondaire monoclonal spécifique de la DIG, pendant 1 heure à 37°C en tampon PBS/1% BSA, ledit anticorps étant biotinylé et disponible dans le commerce (Sigma, réf. B7405), à raison de 0.005 µg/ml (50 µl/puits)
8- 10 lavages en tampon PBST
9- obtention en parallèle d'une molécule d'ADN chimère (DNA Reporter) biotinylée par PCR comme dans l'exemple de la détection de l'hIL-4 susmentionné,
10- préparation en parallèle de streptavidine conjuguée dans le rapport molaire Streptavidine/ADN marqueur biotinylé 0,5/1 (17/34 amoles), laissant ainsi deux sites libres pour la fixation de l'anticorps biotinylé, en tampon PBS/1% BSA
11- ajout de la streptavidine conjuguée dans ladite plaque, le rapport Strept/Ac B choisi est de 0,5/1, soit deux molécules d'Ac fixées par molécule de Streptavidine pendant 30 min à température ambiante (50 µl/puits)
12- 10 lavages en tampon PBST suivis de 3 lavages en H2O
13- PCR quantitative et mesure de la fluorescence durant la phase d'élongation avec un appareil GeneAmp 5700. Afin d'amplifier le ADN marqueur biotinylé décrit ci-dessus, on utilise deux amorces non-biotinylées internes à cet ADN marqueur.
Après 40 cycles d'amplification (10 min à 95°C; 15 sec à 95°C, 1 min à 60°C), on obtient un fragment de 67 pb analysé par le programme GeneAmp SDS software de la firme PE Applied Biosystems.

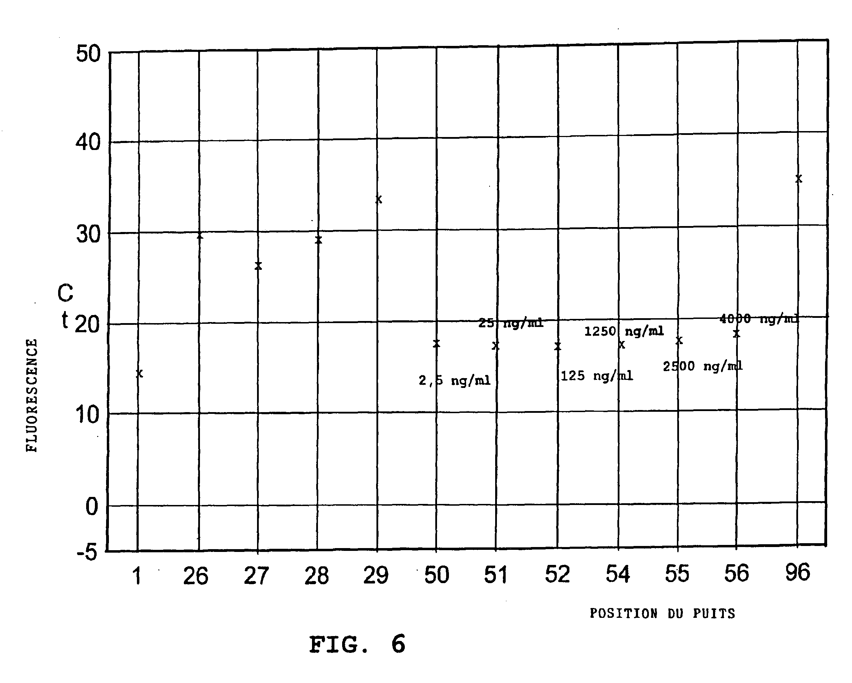

Les résultats sont présentés à la figure 6:
- puits 1: témoin positif (ajout de 12,5 amoles d'ADN Reporter biotinylé avant la PCR quantitative)
- puits 26: témoin sans ajout d'anticorps polyclonal
- puits 27: témoin sans ajout de DNA-DIG
- puits 28: témoin sans ajout d'anticorps monoclonal de détection
- puits 29: témoin sans ajout de complexe streptavidine/ADN ("Reporter") marqueur biotinylé
- puits 50: échantillon testé en présence de 2,5 ng/ml de DNA-DIG
- puits 51: échantillon testé en présence de 25 ng/ml de DNA-DIG
- puits 52: échantillon testé en présence de 125 ng/ml de DNA-DIG
- puits 54: échantillon testé en présence de 1250 ng/ml de DNA-DIG
- puits 55: échantillon testé en présence de 2500 ng/ml de DNA-DIG
- puits 56: échantillon testé en présence de 4000 ng/ml de DNA-DIG
- puits 96: témoin négatif obtenu en l'absence de matrice d'ADN (mélange des réactifs de la PCR et des amorces)

L'analyse de cette figure nous indique que les molécules de DNA-DIG peuvent être détectées au moins de façon qualitative (Ct des échantillons significativement différents des Ct des témoins négatifs).

### EXEMPLE 3 - Le système ligand-PCR : exemple de la BIOTINE

L'invention a également été appliquée au couple streptavidine/biotine (Kd=10⁻¹⁵ M), à titre d'exemple d'application de la PCR quantitative à la détection et la quantification d'éléments ou molécules cibles non antigéniques, par association de ceux-ci avec des "ligands".

On a en effet "coaté" par de la streptavidine, une plaque à 96 puits (5 µg/ml), en ajoutant des rapports molaires variables en ADN biotinylé et en biotine. On observe les variations des signaux obtenus par PCR quantitative. On a ainsi pu déterminer la gamme de concentrations d'ADN biotinylé produisant un signal détectable et exploitable par PCR quantitative. Dans les conditions expérimentales testées, la gamme de concentration optimale (par volume final de 50 µl d'échantillon) est de 10⁻¹⁵ à 10⁻¹⁹ moles (coefficient de corrélation -0,99), avec une préférence pour une concentration de 10⁻¹⁶ moles d'ADN biotinylé /puits.

Avec cette concentration fixe d'ADN biotinylée de 10⁻¹⁶ moles/50 pl, on a réalisé une expérience de compétition entre l'ADN biotinylée et la biotine pour la fixation à la sterptavidine, et on a déterminé une gamme de concentration de biotine s'étendant de 10⁻¹⁴ à 10⁻¹² moles/50 µl, dans des conditions expérimentales comparables.

En conservant une concentration fixe en streptavidine et biotine (respectivement 5 µg/ml et 10⁻¹⁰ moles/50 µl), on a ajouté des concentrations variables en ADN biotinylé, de manière à observer une variation du signal obtenu par PCR quantitative. Pour une gamme de concentration de 10⁻²² à 10⁻¹² moles d'ADN biotinylé on a obtenu une courbe standard présentant un coefficient de corrélation de -0.99.

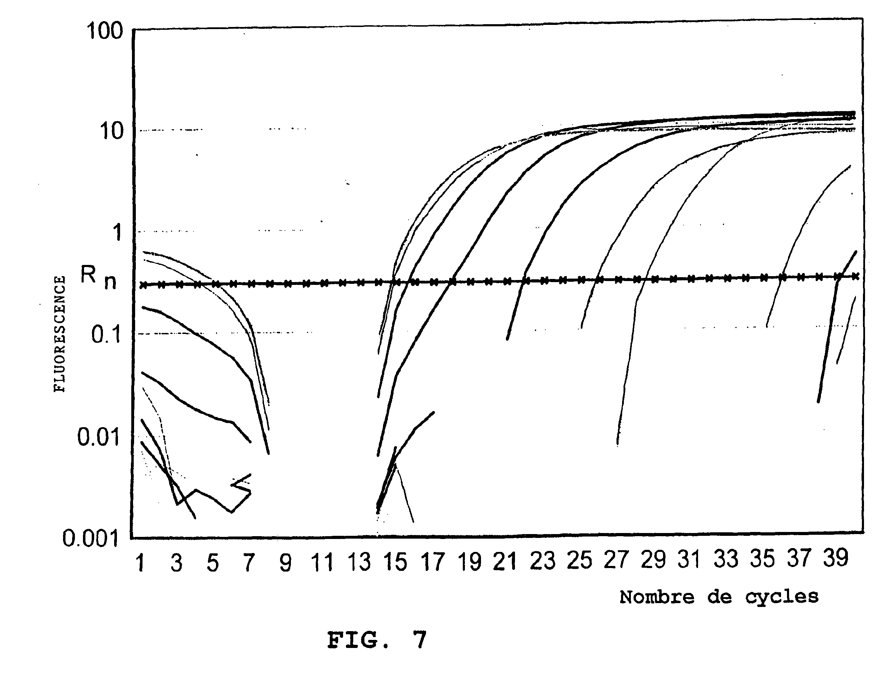

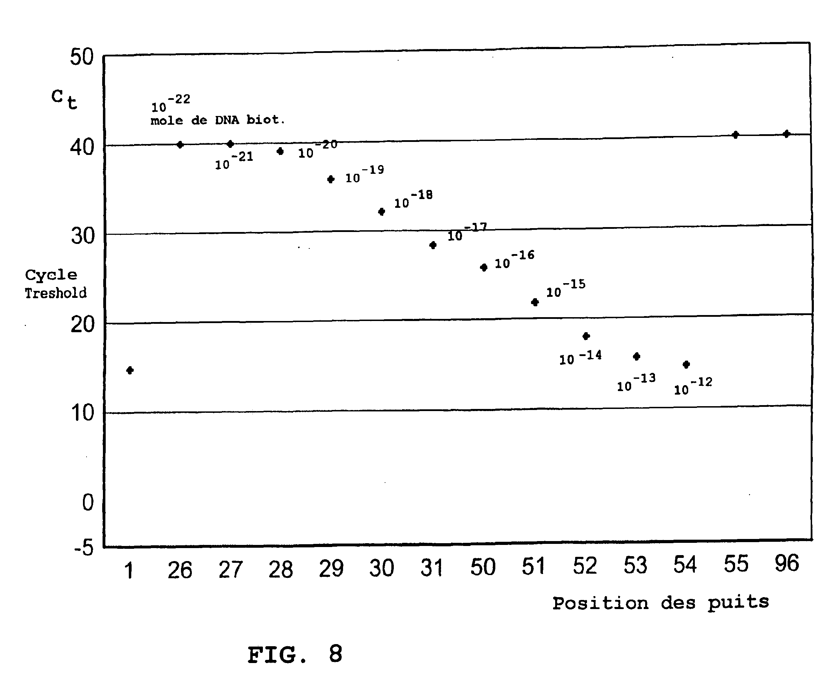

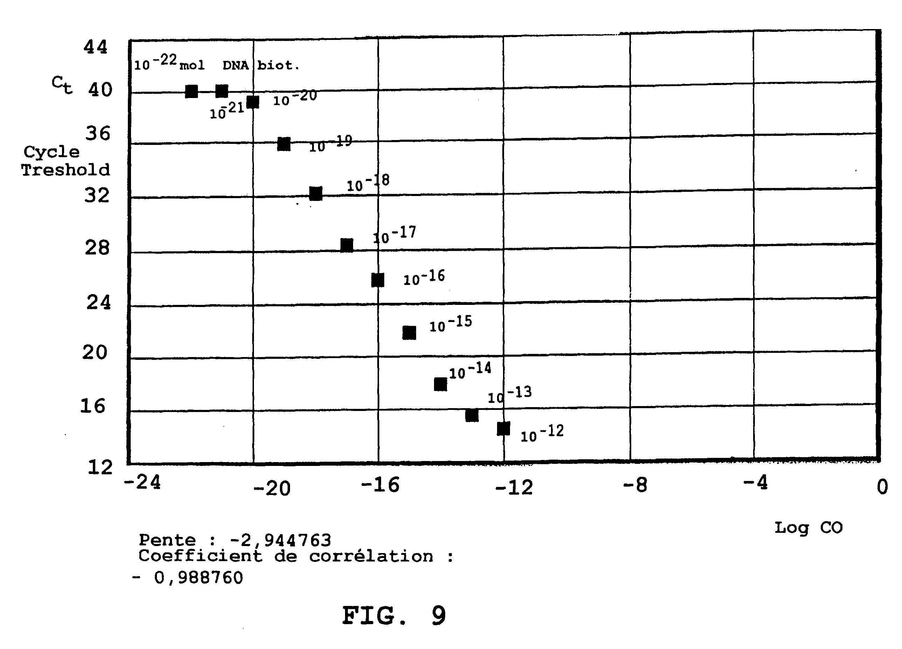

Les figures 7, 8 et 9 représentent respectivement les courbes d'amplification, le graphe des valeurs Ct et la courbe standard associées à cette expérience.

### EXEMPLE 4 : Détection de protéines prions dans des extraits de cerveau par immuno-PCR selon l'invention

Selon un autre aspect de l'invention, l'immuno-PCR quantitative a été rendue applicable à la détection de molécules antigéniques extraites d'organes ou de matrices complexes.

Comme exemple il a été procédé à la détection de la protéine prion native présente dans un extrait total de cerveau de boeuf ayant subi une préparation particulière.

Les étapes de cette partie de l'invention consiste d'abord à trouver les conditions optimales de préparation de l'échantillon en vue de rendre détectable le « pool total de protéines prions » (protéine normale + protéine infectieuse) au sein d'un mélange complexe et de trouver les anticorps adéquats pour réaliser la détection par immuno-PCR quantitative. Parmi les problèmes qu'on peut rencontrer en iPCRq quand il s'agit de détecter un antigène présent dans une matrice complexe :
- problème de PCR croisée avec des oligonucléotides qui risquent de s'hybrider avec d'autres acides nucléiques (ADN, ARN): l'ADN marqueur chimère permet de diminuer les risques mais pas de les éliminer au préalable totalement. Selon cet aspect de l'invention l'ADN marqueur chimère développé pour la détection hIL-4 fonctionne parfaitement et sans générer de signal parasite lors de la détection de protéines prions au sein d'une matrice complexe comme l'extrait de cerveau bovin.
- adaptation d'un protocole de préparation de protéines prions à partir d'extraits de cerveau qui soit compatible avec la détection par immuno-PCR quantitative c'est à dire :
   - traitement spécifique n'utilisant pas de substances inhibant ou interférant avec la Taq polymérase lors de la PCR ;
   - traitement spécifique garantissant la non-activation de tout enzyme issu de la matrice complexe pouvant d'une part dégrader l'antigène à détecter et d'autre part cataboliser les acides nucléiques.

L'invention a donc été d'abord appliquée à la détection de peptides et de protéines prions, plus précisément de séquences polypeptidiques de protéines prions murines et bovines normales et infectieuses.

L'anticorps de capture utilisé est le SAF32 dirigé contre une séquence peptidique correspondant aux résidus 79 à 92 G-G-W-G-Q-P-H-G-G-G-W-G-Q-G-(NH2) de la protéine prion et l'anticorps de détection est le 12F10 dirigé contre une séquence peptidique correspondant aux résidus 126 à 164 G-G-Y-M-L-G-S-A-M-S-R-P-I-I-H-E-G-S-D-Y-E-D-A-Y-Y-R-E-N-M-H-R-Y-P-N-Q-V-Y-Y-R-(NH2) de la protéine prion. Ces anticorps ont été fournis par le Dr. Grassi (CEA, Paris, France). Ils sont utilisés respectivement à une concentration de 10 µg/ml et 0.1 µg/ml.

Le polypeptide PRIO4 cible comprenant les séquences peptiques susmentionnées (fourni par le Dr. Grassi, CEA, Paris, France) est une séquence d'acides aminés synthétique correspondant aux protéines prions et reconnus par les anticorps cités ci-dessus.

Un ELISA a d'abord été mis au point dans le cadre de cette invention. Les données obtenues en iPCRq ont été comparées à celles obtenues par cet ELISA.

Protocole de l'ELISA appliqué au peptide prion.

Le protocole utilisé est celui décrit pour la détection ELISA de protéines prions de boeuf avec les modifications suivantes:
- une digestion de l'extrait protéique par une protéase (protéinase K de préférence mésophile et dans notre cas, dont la température optimale se situe entre 25 et 37°C)
- une inactivation de l'enzyme par chauffage (entre 50 et 100°C) de préférence entre 90 et 100°C en solution aqueuse, afin d'éviter la digestion par la protéinase K des constituants protéiques intervenant dans l'ELISA (anticorps, enzymes tels que la peroxydase ou la phosphatase).

Les étapes du protocole sont les suivantes .
1- Coatage de l'anticorps monoclonal de capture SAF32 dans une plaque à 96 puits de type ELISA (Nunc) (100 µl/puits), en tampon de coatage (NaHCO₃ 0.05M, Na₂CO₃ 0.05M, pH 9.4) à 4°C pendant une nuit, à une concentration de 10 µg/ml
2- 5 lavages en solution de lavage (SLT)(NaCl 0.15M, Tween 20 0.1%, pH 7.4) (300 µl/puits)
3- Saturation des sites non saturés par l'anticorps de capture en tampon de saturation (NaCl 0.14M, Na₂HPO₄.2H₂O 8 mM, KH₂PO4 1.5 mM, KCl 2.7 mM, BSA 3%, pH 7.4) pendant 2 heures à température ambiante (300 µl/puits)
4- 5 lavages en solution de lavage (SLT)
5- Ajout d'un peptide « Prions » (ici : peptide PRI04) au tampon de dilution (NaCl 0.14M, Na₂HPO₄.2H₂O 8 mM, KH₂PO₄ 1.5 mM, KCl 2.7 mM, BSA 1%, Tween 20 0.1%, pH 7.4), aux concentrations suivantes: 100µg/ml, 1ng/ml, 10ng/ml, 0.1µg/ml, 1µg/ml, 10 µg/ml, 100 µg/ml, pendant 1 heures à température ambiante (100 µl/puits).
6- 5 lavages en solution de lavage (SLT)
7- Ajout d'un anticorps monoclonal biotinylé de détection 12F10 à une concentration de 0.1 µg/ml en tampon de dilution, pendant 1 heure à température ambiante (100 µl/puits).
8- 5 lavages en solution de lavage (SLT)
9- Ajout de streptavidine conjuguée à la peroxydase (dilué 750 fois en tampon de dilution) pendant 30 minutes à température ambiante (100 µl/puits).
10- 5 lavages en solution de lavage (SLT)
11- Ajout de TMB (BD-Pharmingen, substrat de la peroxydase) pendant 30 minutes à température ambiante (100 µl/puits) et à l'abri de la lumière.
12- Arrêt de la réaction par du H₂SO₄ 2N (50 µl/puits)
13- Lecture de l'absorbance à 450 nm au spectrophotomètre (Labsystems Multiskans MS).

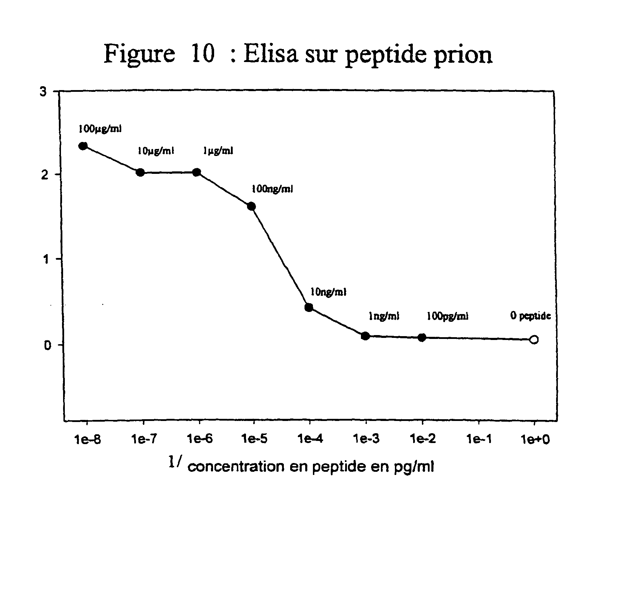

Après avoir soustrait la valeur moyenne d'absorbance obtenue pour les témoins négatifs (correspondant à l'expérience réalisée en l'absence d'anticorps de capture, d'antigènes, d'anticorps de détection ou de Streptavidine/peroxydase) de celle des échantillons, nous avons reporté ces valeurs (A_{450 nm}) en fonction de la concentration du peptide. Les résultats sont présentés à la figure 10.

Les résultats obtenus à la suite de cette expérience montrent que la gamme de concentrations du peptide prion détectables par ELISA est située entre 1ng/ml et 100gg/ml. En tenant compte de ces résultats nous avons réalisé une expérience d'iPCRq sur le même peptide prion.

Protocole de l'iPCRq appliqué au peptide prion
1- Coatage de l'anticorps monoclonal de capture SAF32 dans une plaque optique à 96 puits de la firme PE Applied Biosystems(50 µl/puits), en tampon de coatage (NaHCO₃ 0.05M, Na₂CO₃ 0.05M, pH 9.4) une nuit à 4°C, à une concentration de 10 µg/ml
2- 4 lavages en solution de lavage (SLT) (NaCl 0.15M, Tween 20 0.1%, pH 7.4) (300 µl/puits)
3- Saturation des sites non saturés par l'anticorps de capture en tampon de saturation (NaCl 0.14M, Na₂HPO₄.2H₂O 8 mM, kH₂PO₄ 1.5 mM, KCl 2.7 mM, BSA 3%, pH 7.4) pendant 2 heures à température ambiante (300 µl/puits)
4- 5 lavages en solution de lavage (SLT)
5- Ajout du peptide PRI04 en tampon de dilution (NaCl 0.14M, Na₂HPO₄.2H₂O 8 mM, KH₂PO₄ 1.5 mM, KC1 2.7 mM, BSA 0.5%, Tween 20 0.1%, pH 7.4), aux concentrations suivantes: 1pg/ml, 10pg/ml, 100pg/ml, 1ng/ml, 10ng/ml, 0.1µg/ml, 1µg/ml puis ajout d'un anticorps monoclonal biotinylé de détection 12F10 (25 µl/puits), à une concentration de 0.1 µg/ml en tampon de dilution, pendant 2 heures à température ambiante, sous une agitation constante de 280 rpm
6- 10 lavages en solution de lavage (SLT)
7- Ajout de complexes S/Db en tampon de dilution pendant 30 minutes à température ambiante (50 µl/puits), aux concentrations suivantes (rapports molaires 0.5/1): 17 amoles/34 amoles.
8- 10 lavages en solution de lavage(SLT)-5 lavages en H₂O
9- 40 cycles de Q-PCR à l'aide du GeneAmp 5700 Sequence Detection System de la firme PE Biosystems.

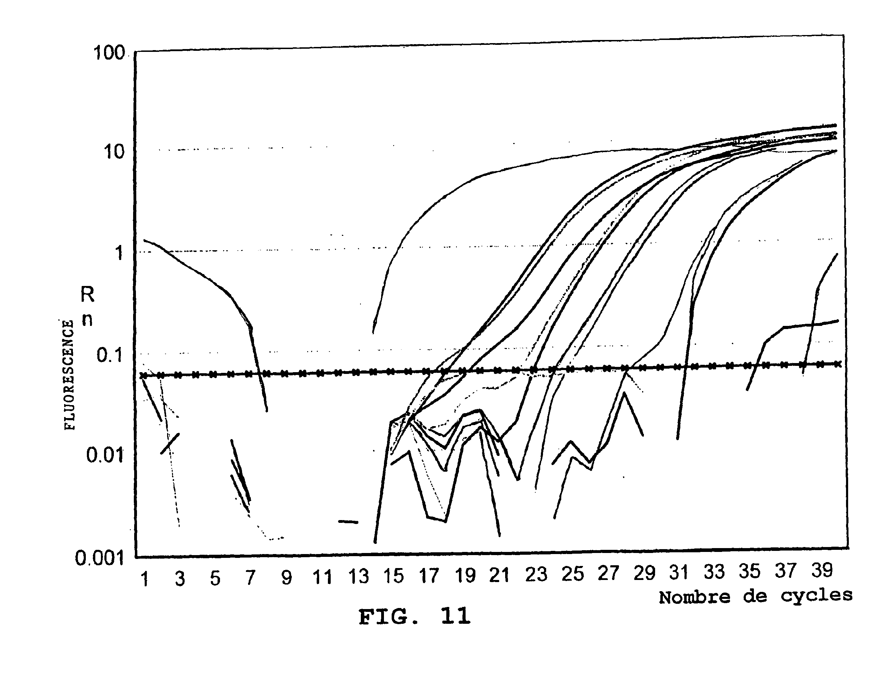

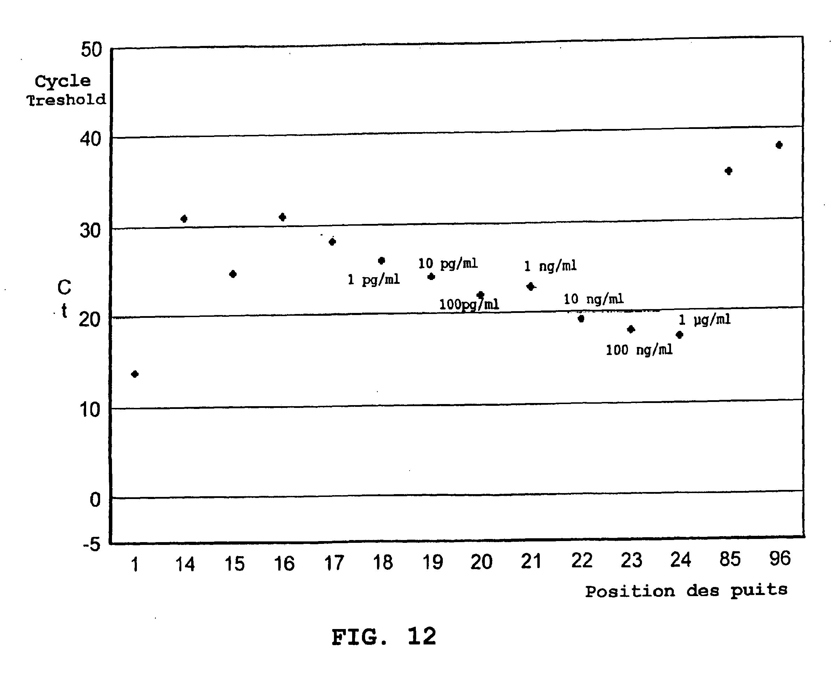

Les courbes d'amplification représentant la fluorescence des échantillons en fonction du nombre de cycles sont présentés à la figure 11, les Ct reportés en fonction de la position de l'échantillon dans la plaque sont illustrés à la figure 12.

Les résultats obtenus lors de cette expérience et présentés à la figure 12 montrent que la gamme de concentrations détectables du peptide prion est située entre 10pg/ml et 1µg/ml ou plus. La sensibilité atteinte (10pg/ml) s'est révélée supérieure d'un facteur d'au moins 100 à celui de l'ELISA dont la limite de détection a été déterminé à 1 ng/ml.

Protocole de l'ELISA appliqué à l'extrait protéique prion bovin.

L'extrait protéique (environs 6.9 mg/ml) à été obtenu à partir de 4 g de cerveau de boeuf suivant la procédure décrite par le Dr Grassi (CEA, Paris, France ; Rodolfo,K et al article soumis).

Le protocole expérimental d'ELISA est identique à celui décrit pour le peptide prion excepté au niveau du point 5 pour les concentrations de protéines d'un extrait de boeuf :
- ajout de l'extrait protéique en tampon de dilution (NaCl 0.14M, Na₂HPO₄.2H₂O 8 mM, KH₂PO₄ 1.5 mM, KCl 2.7 mM, BSA 1%, Tween 20 0.1%, pH 7.4), aux concentrations suivantes: 5ng/ml, 50ng/ml, 500ng/ml, 5 µg/ml, 50 µg/ml, 500µg/ml, 5mg/ml pendant 1 heure à température ambiante (100 µl/puits).

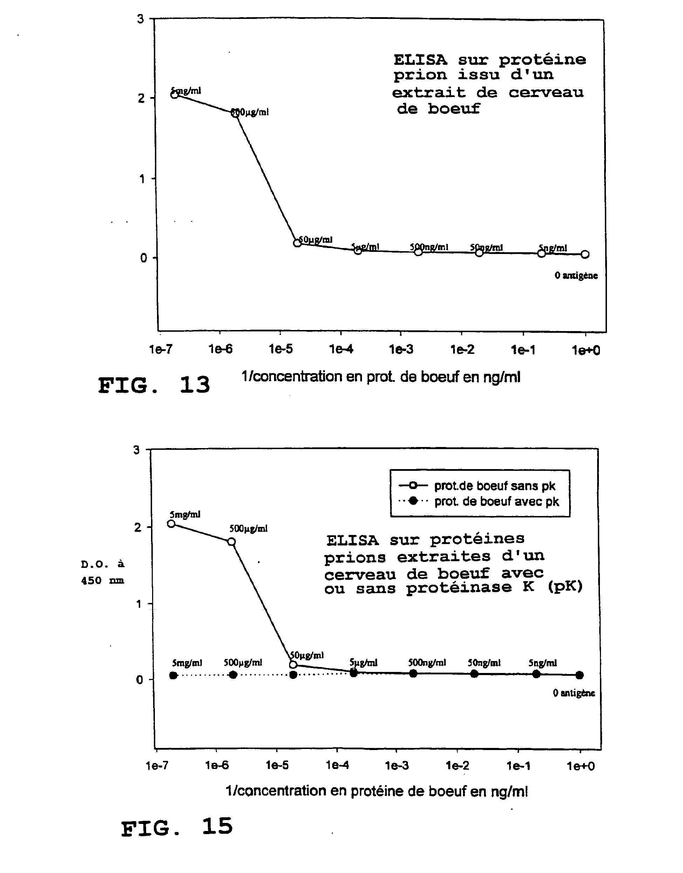

Les résultats du test ELISA présentés à la figure 13 où l'absorbance à 450nm est portée en fonction de la concentration de l'extrait protéique montrent que la limite de détection du test est de l'ordre de 50 µg/ml jusqu'à 5 ng/ml.

Protocole d'iPCRq-prion appliqué à l'extrait protéique bovin.

Le protocole expérimental est identique à celui décrit pour le peptide prion excepté pour le point 5- suivant : 5- Ajout de l'extrait protéique en tampon de dilution (NaCl 0.14M, Na₂HPO₄.2H₂O 8 mM, KH₂PO₄ 1.5 mM, KCl 2.7 mM, BSA 1%, Tween 20 0.1%, pH 7.4), aux concentrations suivantes: 5pg/ml, 50pg/ml, 500pg/ml, 5ng/ml, 50ng/ml, 500ng/ml, 5µg/ ml puis ajout d'un anticorps monoclonal biotinylé de détection 12F10 (25 µl/puits), à une concentration de 0.1 µg/ml en tampon de dilution, pendant 2 heures à température ambiante, sous une agitation constante de 280 rpm.

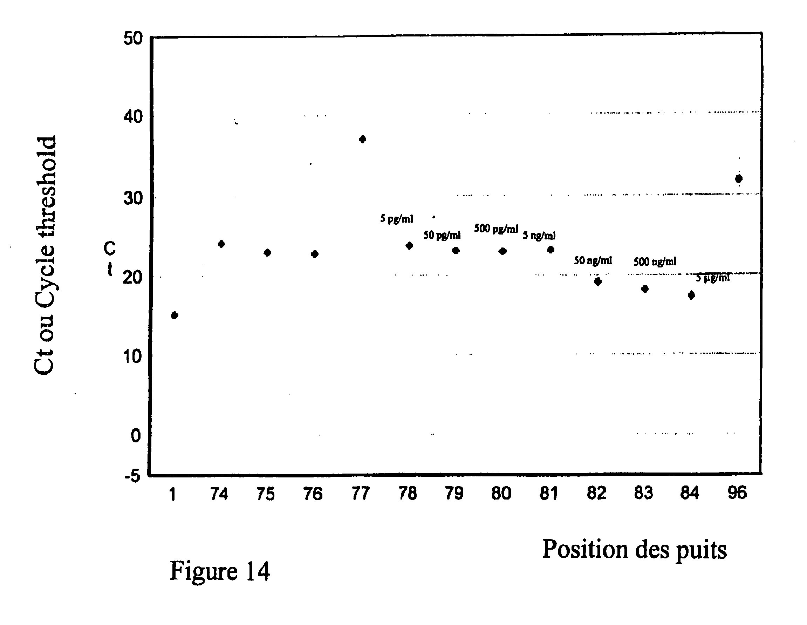

Les Ct des échantillons calculés et portés en fonction de la position de l'échantillon (fig. 14) montrent une amélioration du seuil de détection par rapport à l'ELISA.

De manière tout à fait générale, on comprendra que l'invention peut s'appliquer pour la détection, quantitative d'un des composants d'un couple de composants présentant une affinité l'un pour l'autre (p.e. hormone - récepteur, anticorps - antigène, molécule organique cible-ligand, cellule A- cellule B, enzyme - substrat, protéine-acide nucléique, acide nucléique A-nucléique B). La seule condition est qu'un des composants doit pouvoir être conjugué ou couplé à un acide nucléique "reporter" marqueur qui est constitué d'une séquence ADN chimère et l'autre composant doit pouvoir être fixé, directement ou indirectement, sur une paroi. par des cycles de transcription-réverse, remplacant ainsi la PCR classique.

On notera qu'un ADN marqueur chimère du type utilisé dans le cadre de la présente invention peut s'avérer utile dans des tests de détection ou de quantification d'éléments à doser en particulier des antigènes, plus particulièrement des microorganismes.

De façon générale, la détection par ELISA direct ou indirect ou par Quick-ELISA direct ou indirect ou par ELISA (Fluorescent Linked Immunosorbent Assay) direct ou indirect d'élément antigénique est transposable à l'immuno-PCR selon l'invention

L'invention est définie par les revendications qui suivent.

### Liste des séquences

SEQ ID NO :1
   Type de séquence : nucléotide
   Longueur de la séquence : 246 bp
   Nombre de brins : double, et représenté sous forme simple
   Configuration : linéaire 
5'
TATCCAATCCATTCCAGGCCCTGTCCAGGCCTCTGTTTCACCCAGTTTATACCGTAGCTTGT
GAATGTATATCCAGAAGCCTTGCAGGACATCTTCACTGAGGACAAGGGCGAATTCTGCAGA
TATCCATCACACTGGCGGCCGCTCGAGCATGCATCTAGAGGGCCCAATTCGCCCTATAGTGAG
TCGTATTACAATTCACTGGCCGTCGTTTTACAACGTCGTGACTGGGAAAACCCTGGCGTTA
3'
SEQ ID NO : 2
   Type de séquence : nucléotide
   Longueur de la séquence : 21 b
   Nombre de brins : simple
   Configuration : linéaire
   5'Biotine-TAT CCA ATÇ CAT TCC AGG CCC 3'
SEQ ID NO : 3
   Type de séquence : nucléotide
   Longueur de la séquence : 21 b
   Nombre de brins : simple
   Configuration : linéaire
   5' TAA CGC CAG GGT TTT CCC AGT 3'
SEQ ID NO : 4
   Type de séquence : nucléotide
   Longueur de la séquence : 22 b
   Nombre de brins : simple
   Configuration : linéaire
   5' GAA GCC TTG CAG GAC ATC TTC A 3'
SEQ ID NO : 5
   Type de séquence : nucléotide
   Longueur de la séquence : 20 b
   Nombre de brins : simple
   Configuration : linéaire
   5' GCC GCC AGT GTG ATG GAT AT 3'

## Revendications

1. Procédé de détection ou de dosage d'un élément microbiologique A
a. comprenant les étapes suivantes:
- une étape de fixation dudit élément A, directement ou par l'intermédiaire d'un agent de capture, à une ou des surfaces à l'intérieur du ou des récipients d'un appareil de mesure PCR,
- une étape d'association audit élément A d'un ligand pour cet élément A sous la forme d'un système de couplage à un acide nucléique marqueur, et
- une étape d'amplification de l'acide nucléique marqueur,
b. **caractérisé en ce que** des mesures successives de l'appareil de mesure PCR sont effectuées de manière quantitative durant la phase d'amplification de l'acide nucléique marqueur avec un appareil de PCR apte à mesurer, pendant la phase d'amplification, un signal représentatif d'une quantité d'amplicons spécifiques du fragment d'ADN, ce dernier étant marqué au cours de la PCR par un agent fixé à l'acide nucléique, excitable par un rayonnement électromagnétique, et **en ce que**
l'acide nucléique marqueur est constitué d'une séquence ADN chimère.

2. Procédé selon la revendication 1 **caractérisé en ce que** les surfaces de fixation sont des parois intérieures de tubes PCR en plastique.

3. Procédé selon n'importe laquelle des revendications 1 à 2 **caractérisé en ce qu'**il s'agit d'un procédé de détection et/ou de dosage par immuno-PCR d'un élément antigénique, les mesures durant la phase d'amplification étant effectuées en présence des anticorps et de l'élément antigénique à doser, qui ont été fixés sur des surfaces à l'intérieur du ou des récipients de l'appareil de mesure.

4. Procédé selon la revendication 3 dans lequel l'élément antigénique est une protéine, un antigène de surface, un carbohydrate, une hormone, une bactérie, un ligand, un récepteur, un composé inorganique, un composé organique, en particulier un composé aromatique, un antigène tumoral, un peptide, une cytokine ou un acide nucléique.

5. Procédé selon n'importe laquelle des revendications précédentes comprenant les étapes suivantes:
- on couvre une surface d'un récipient ou d'une bille avec un anticorps A, ou d'un fragment A d'immunoglobuline Fc, Fab, Fv naturelle ou de synthèse
- on ajoute une solution de l'élément antigénique comportant au moins deux sites antigéniques dont l'un au moins complémentaire audit anticorps A, ou fragment A
- on associe un fragment de ADN marqueur à au moins un second anticorps B, ou d'un fragment B d'immunoglobuline Fc, Fab ayant une affinité sélective pour l'élément antigénique à détecter pour obtenir un conjugué
- on ajoute ledit conjugué à la solution
- on réalise une PCR sur les fragments d'ADN marqueurs ainsi immobilisés sur ladite surface
- on détecte et mesure l'amplification, par exemple par fluorescence.

6. Procédé selon n'importe laquelle des revendications 3 à 5 dans lequel l'anticorps ou molécule dérivée B et l'ADN marqueur sont tous deux biotinylés et sont associés par l'intermédiaire d'une molécule synthétique ou naturelle présentant une affinité élevée pour la biotine.

7. Procédé selon n'importe laquelle des revendications 5 à 6 dans lequel l'anticorps B est de préférence monoclonal et est directement lié par une liaison covalente au fragment d'ADN marqueur.

8. Utilisation d'un appareil de PCR quantitative pour le dosage d'antigènes par la technique PCR de n'importe laquelle des revendications 1 à 2 ou par la technique d'immuno-PCR d'une des revendications 3 à 7.

9. Procédé selon n'importe laquelle des revendications 1 à 8 **caractérisé en ce que** les surfaces de fixation sont des parois intérieures de tubes PCR en polypropylène.

10. Procédé selon n'importe laquelle des revendications précédentes dans lequel l'élément à doser est présent dans un extrait de matrice complexe organique ou environnementale.

11. Procédé selon n'importe laquelle des revendications précédentes dans lequel l'élément à doser est un ou des polypeptides prions infectueux.

12. Procédé selon n'importe laquelle des revendications précédentes **caractérisé en ce que** l'acide nucléique marqueur est de séquence SEQ ID NO : 1.

13. Acide nucléique de séquence SEQ ID NO: 1.

14. Acide nucléique selon la revendication 13 qui est biotynilé.

15. Acide nucléique choisi parmi le groupe constitué des acides nucléiques de séquence SEQ ID NO :2, SEQ ID NO : 3, SEQ ID NO 4, SEQ ID NO : 5 .

## Claims

1. A process for detecting or for assaying an element A
a. including the following steps:
- a step in which element A is bound, directly or through a capture agent, to one or more surfaces inside one or more containers of a PCR measuring apparatus, and
- a step in which said element A is associated to a ligand for this element A in the form of a system of coupling to a nucleic acid marker and
- a step in which the marker nucleic acid is amplified,
b. **characterized in that** successive measurements of the PCR measuring apparatus are performed during marker nucleic acid amplification, and **in that** the marker DNA fragment consists of a chimeric sequence.

2. A process according to claim 1 **characterized in that** the binding surfaces are the inner walls of plastic PCR tubes.

3. A process according to any of the claims 1 to 2 **characterized in that** it is a process for the detection and/or the assaying by immuno-PCR of an antigenic element, the measurements during the amplification stage being performed in the presence of the antibodies and of the antigenic elements to be assayed, which have been fixed on surfaces at the inside of the container(s) of the measuring apparatus.

4. Process according to claim 3 wherein the antigenic element is a protein, a surface antigen, a carbohydrate, a hormone, a bacterium, a ligand , a receptor, an inorganic compound, an organic compound, more particularly an aromatic compound, a tumor antigen, a peptide, a cytokine or a nucleic acid.

5. A process as claimed in any preceding claim, including the following steps:
- coating a surface of a container or bead with a solution of an antibody A, or a natural or synthetic immunoglobulin fragment A (Fc, Fab, Fv)
- adding a solution of the antigenic element possessing at least two antigenic sites, at least one of these sites being complementary to said antibody A or fragment A
- associating a marker DNA fragment with at least one second antibody B, or of a fragment B of immunoglobulin Fc, Fab, having a selective affinity for the antigenic element to be detected in order to obtain a conjugate
- adding said conjugate to the solution
- performing a PCR on the DNA marker fragments thus immobilized on said surface
- detecting and measuring amplification, for instance by fluorescence.

6. A process according to any of the claims 3 to 5, wherein the antibody or derived molecule B and the marker DNA are both biotinylated and are associated with each other through a synthetic or natural molecule having a high affinity for biotin.

7. A process according to any of the claims 5 to 6, wherein the antibody B is preferably monoclonal and is directly linked by a covalent bond to the marker DNA fragment.

8. Use of a quantitative PCR apparatus for assaying antigens by the PCR technique of any of the claims 1 and 2 or by the immuno-PCR technique of any one of the claims 3 to 7.

9. A process according to any of the claims 1 to 8 **characterized in that** the binding surfaces are the inner walls of polypropylene PCR tubes.

10. A process according to any of the preceding claims wherein the element to be assayed is present in an extract of a complex organic or environmental matrix.

11. A process according to any of the preceding claims wherein the element to be assayed is one or more infectious prion polypeptide(s).

12. A process according to any of the preceding claims **characterized in that** the marker nucleic acid has the sequence SEQ ID NO: 1.

13. A nucleic acid which has the sequence SEQ ID NO : 1.

14. A nucleic acid of claim 13 which is biotinylated.

15. A nucleic acid chosen from the group consisting of nucleic acids with the sequences SEQ ID NO :2, SEQ ID NO : 3, SEQ ID NO 4, SEQ ID NO 5 .

## Patentansprüche

1. Verfahren zur Detektion oder Dosierung eines mikrobiologischen Elements A
a. umfassend die folgenden Schritte:
- einen Schritt der Fixierung des Elements A, direkt oder vermittels eines Einfangmittels, an eine Fläche oder an Flächen im Inneren von einem Behälter oder von Behältern eines PCR-Messgerätes,
- einen Schritt der Assoziation des Elements A mit einem Liganden für dieses Element A in Form eines Systems der Kopplung an eine Marker-Nukleinsäure, und
- einen Schritt der Amplifikation der Marker-Nukleinsäure
b. **dadurch gekennzeichnet, dass** nachfolgende Messungen des PCR-Messgerätes in quantitativer Weise während der Phase der Amplifikation der Marker-Nukleinsäure mit einem PCR-Gerät durchgeführt werden, das in der Lage ist, während der Phase der Amplifikation ein Signal zu messen, das repräsentativ ist für eine Menge Amplikons, die für das DNA-Fragment spezifisch sind, wobei letzteres durch ein im Verlauf der PCR an der Nukleinsäure fixiertes Mittel markiert ist, das durch elektromagnetische Strahlung anregbar ist, und **dadurch**, dass die Marker-Nukleinsäure aus einer chimären DNA-Sequenz besteht.

2. Verfahren nach Anspruch 1, **dadurch gekennzeichnet, dass** die Fixierungsflächen Innenwände von Kunststoff-PCR-Röhrchen sind.

3. Verfahren nach einem der Ansprüche 1 bis 2, **dadurch gekennzeichnet, dass** es sich um ein Verfahren zur Detektion und/oder Dosierung eines antigenischen Elements durch Immun-PCR handelt, wobei die Messungen während der Amplifikationsphase in Gegenwart von Antikörpern und des zu dosierenden antigenischen Elements, die auf Flächen im Inneren des Behälters oder der Behälter des Messgerätes fixiert sind, durchgeführt werden.

4. Verfahren nach Anspruch 3, bei dem das antigenische Element ein Protein, ein Oberflächenantigen, ein Kohlenwasserstoff, ein Hormon, ein Bakterium, ein Ligand, ein Rezeptor, eine anorganische Zusammensetzung, eine organische Zusammensetzung, insbesondere eine aromatische Zusammensetzung, ein Tumorantigen, ein Peptid, ein Zytokin oder eine Nukleinsäure ist.

5. Verfahren nach einem der vorhergehenden Ansprüche, umfassend die folgenden Schritte:
- man bedeckt eine Fläche eines Behälters oder einer Kugel mit einem natürlichen oder synthetischen Antikörper A oder Fragment A von Immunglobulin Fc, Fab, Fv
- man fügt eine Lösung des antigenischen Elements hinzu, das mindestens zwei antigenische Stellen beinhaltet, von denen mindestens eine zu dem Antikörper A oder dem Fragment A komplementär ist
- man assoziiert ein Marker-DNA-Fragment mit mindestens einem zweiten eine selektive Affinität für das zu detektierende antigenische Element aufweisenden Antikörper B oder Fragment B von Immunglobulin Fc, Fab, um ein Konjugat zu erhalten
- man fügt das Konjugat zu der Lösung hinzu
- man führt eine PCR für die Marker-DNA-Fragmente durch, die auf diese Weise auf der Fläche immobilisiert sind
- man detektiert und misst die Amplifikation, beispielsweise durch Fluoreszenz

6. Verfahren nach einem der Ansprüche 3 bis 5, bei dem der Antikörper oder das Molekülderivat B und die Marker-DNA beide biotinyliert sind und vermittels eines synthetischen oder natürlichen Moleküls assoziiert sind, das eine erhöhte Affinität für Biotin aufweist.

7. Verfahren nach einem der Ansprüche 5 bis 6, bei dem der Antikörper B vorzugsweise monoklonal ist und direkt durch eine kovalente Bindung mit dem Marker-DNA-Fragment verbunden ist.

8. Verwendung eines quantitativen PCR-Gerätes zur Dosierung von Antigenen durch eine PCR-Technik nach irgendeinem der Ansprüche 1 bis 2 oder durch eine Immun-PCR-Technik nach einem der Ansprüche 3 bis 7.

9. Verfahren nach einem der Ansprüche 1 bis 8, **dadurch gekennzeichnet, dass** die Fixierungsflächen Innenwände von PCR-Röhrchen aus Polypropylen sind.

10. Verfahren nach einem der vorhergehenden Ansprüche, bei dem das zu dosierende Element in einem Extrakt einer komplexen organischen oder Umweltmatrix vorliegt.

11. Verfahren nach einem der vorhergehenden Ansprüche, bei dem das zu dosierende Element ein oder mehrere infektiöse Prion-Polypeptide ist.

12. Verfahren nach einem der vorhergehenden Ansprüche, **dadurch gekennzeichnet, dass** die Marker-Nukleinsäure die Sequenz der SEQ ID NO: 1 aufweist.

13. Nukleinsäure der Sequenz SEQ ID NO: 1.

14. Nukleinsäure nach Anspruch 13, die biotinyliert ist.

15. Nukleinsäure, ausgewählt aus der Gruppe bestehend aus den Nukleinsäuren der Sequenz SEQ ID NO: 2, SEQ ID NO: 3, SEQ ID NO: 4, SEQ ID NO: 5.
